# FASCICULE DE BREVET EUROPEEN

(11) **EP 0 623 344 B1**
(45) Date de publication et mention de la délivrance du brevet: **26.07.2000**
(21) Numéro de dépôt: 94400949.7
(22) Date de dépôt: 02.05.1994
(51) Int. Cl.: A61K 31/135

(54) **Utilisation de la Sélégiline en médecine vetérinaire**
Anwendung von Selegilin in Tiermedizin
Use of Selegiline in vetenary medicine

(30) Priorité: 04.05.1993 FR 9305305; 22.12.1993 FR 9315495
(43) Date de publication de la demande: 09.11.1994
(73) Titulaire: CEVA SANTE ANIMALE, 33500 Libourne (FR)
(72) Inventeur: Filhol, Marie Sophie, F-33400 Talence (FR); Pobel, Thierry, F-33870 Vayres (FR)
(74) Mandataire: Le Guen, Gérard

(56) Documents cités:
- EP-A- 0 473 252
- WO-A-88/04552
- WO-A-90/01928
- WO-A-91/18592
- WO-A-92/17169
- US-A- 5 192 808
- CHEMICAL ABSTRACTS, vol. 117, no. 25, 21 Décembre 1992, Columbus, Ohio, US; abstract no. 245502c, & PHARMACOL. BIOCHEM. BEHAV. vol. 43, no. 3 , 1992 pages 749 - 757 E. HEAD ET AL. 'CHANGES IN SPONTANEOUS BEHAVIOR IN THE DOG FOLLOWING ORAL ADMINISTRATION OF L-DEPRENYL'

## Description

La présente invention concerne l'utilisation de la sélégiline pour la préparation de médicaments utiles pour traiter les troubles du comportement avec altération de l'humeur des animaux de compagnie, chiens et chats.

La sélégiline, largement utilisée chez l'homme en association avec la lévodopa dans le traitement du parkinsonisme, a aussi été étudiée dans le traitement des maladies de la sénescence, comme la maladie d'Alzheimer (cf. par ex. EP-A-0406488, -0451484).

Elle a été récemment décrite dans EP-A-0473252 comme utilisable pour retarder les effets physiologiques néfastes de l'âge sur les organismes animaux, en particulier sur les reins, les glandes et le système immunitaire et pour limiter la détérioration des capacités d'apprentissage et de mémorisation des chats et chiens âgés ; la plupart de ces activités qui ne peuvent être constatées qu'après une administration prolongée aux animaux adultes restent à démontrer en clinique.

D'autres expérimentateurs ont observé chez certains rats, une prolongation sensible de la durée de vie lorsque la sélégiline leur est administrée régulièrement à la dose de 0,25 mg/kg après 23 mois alors que leur durée de vie moyenne est de 35 mois ; J. Knoll observe dans Mechanisms of Ageing and Development 46 237-262 (1988) qu'à cette prolongation s'ajoute une amélioration de l'activité sexuelle des animaux et que celles-ci pourraient être dues à une action de la sélégiline sur les neurones dopaminergiques du corps strié.

Chemical Abstracts, vol. 117, n° 25, 21/12/1992, concerne une étude sur l'influence de la sélégiline sur le comportement du chien. Cette étude porte sur des animaux sains, d'âges variés (cf. p. 751, collone de gauche).

Plus précisément, ce document révèle que l'administration par voie orale de sélégiline à des doses de 2 à 5 mg/kg a pour effet d'augmenter les stéréotypies : la locomotion des chiens femelles est notamment augmentée, cependant leur activité est désordonnée, les chiens décrivant des cercles en continu.

Les autres effets observés sont une perte de l'intérêt du chien pour son environnement et une diminution du besoin d'uriner.

On a maintenant constaté que l'administration de sélégiline aux animaux de compagnie permettait d'améliorer très rapidement et de régulariser en quelques semaines le comportement de ceux-ci qui est parfois suffisamment insupportable pour le maître pour qu'il se sépare de son animal, l'euthanasie étant fréquemment utilisée à l'heure actuelle.

Les troubles du comportement avec altération de l'humeur se caractérisent par une succession de phases d'hyperactivité incoordonnée avec insomnies, plus ou moins accompagnée d'agressivité et de phases d'apathie avec des difficultés locomotrices, de l'hypersomnie et de l'hyporexie. On traite, actuellement, les animaux présentant de tels troubles en leur administrant des médicaments neuroleptiques , tels que des benzodiazépines ou des benzamides, dont l'efficacité, qui n'apparait que lentement, est souvent accompagnée d'une activité hypnotique.

L'invention concerne, en conséquence, l'utilisation de la sélégiline, de son racémique, de l'isomère lévogyre ou de leurs mélanges en proportions quelconques et de leurs sels pharmaceutiquement acceptables pour la préparation d'une composition pharmaceutique destinée au traitement des troubles du comportement avec altération de l'humeur chez le chien ou le chat,
- par voie orale à une dose journalière de 0,25 à 1 mg/kg de poids corporel de l'animal ; ou
- par voie sous-cutanée à une dose journalière de 0,25 à 4 mg/kg de poids corporel de l'animal.

Selon un mode de réalisation préféré de l'invention, on utilise à titre de principe actif, du chlorhydrate de sélégiline.

Cette composition peut se présenter sous l'une des formes pharmaceutiques généralement utilisées en médecine vétérinaire, de préférence pour une administration par voie orale.

Dans ce cas, la dose journalière de sélégiline, HCl pourra aller de 0,25 mg à 1 mg/kg de poids corporel de l'animal et de préférence voisine de 0,5 mg/kg; mais ces limites pourront être dépassées en fonction de l'âge de l'animal ou de l'intensité des symptômes.

Comme les animaux peuvent être de poids très différents, on pourra présenter le médicament par voie orale dans une forme facile à diviser, par exemple en comprimés sécables, en 2 ou 4 morceaux, à la main. On l'administrera en général par voie orale le matin à jeun.

L'administration peut aussi être effectuée en sous-cutanée, notamment chez le chat, a la dose journalière allant de 0,25 à 4 mg/kg de sélégiline, HCl.

Le traitement sera de préférence maintenu quelques jours après la disparition des symptômes; néanmoins, il apparait inutile de le prolonger au-delà de 8 semaines; en général il durera de 3 à 6 semaines.

Un mode d'administration particulièrement préféré est l'administration par voie orale.

Les compositions pharmaceutiques conformes à l'invention seront préparées de façon classique avec les excipients couramment utilisés dans le domaine pour obtenir des compositions solides, liquides ou pâteuses, notamment des comprimés, des gélules, ou des poudres; par exemple des comprimés divisibles en 4, contenant 4 mg de sélégiline, HCl pour environ 200 mg d'excipients, seront préconisés pour les animaux de 2 à 8 kg alors que ceux comprenant 10 mg de sélégiline, HCl pour 500 mg conviendront aux animaux de 9 à 20 kg. Pour l'administration sous-cutanée, on utilisera des solutions aqueuses contenant 10 mg de sélégiline, HCl par ml, le volume injecté étant de 0,5 à 2 ml selon le poids de l'animal.

On pourra se référer aux ouvrages bien connus du galéniste tels que The theory and pratice of Industrial Pharmacy - L. Lachman & al.- Lea & Febiger (1986) - Philadelphia, ou Pharmacie galénique A. Denoël, Fr. Jaminet.- Presses Universitaire de Liège (1971).

### 1. Etude clinique préliminaire

Dans ce qui suit, on décrit les observations cliniques effectuées sur des chiens de diverses races, mâles ou femelles, ayant plus de 2 mois, dont les troubles du comportement se traduisent par plusieurs symptômes dans la liste suivante :
- boulimie avec ou sans régurgitation et réingestion ou au contraire anorexie ou dysorexie
- eudipsie, polydipsie ou mâchonne l'eau sans l'avaler
- rituels de mordillement ou léchages anormaux,
- malpropreté urinaire ou fécale
- sommeil perturbé

L'animal perturbé volera des objets ou refusera de les rendre, ne répondra pas aux ordres et aux réprimandes, réagira mal, y compris par l'indifférence, aux changements et aux situations oppressantes.

Dans le tableau I suivant sont résumées les conclusions des observations pour les chiens auquels on a administré des comprimés contenant 10 mg de sélégiline chaque jour, le matin à jeun à la dose indiquée.

**Tableau I**

| **Animal** | **Dose** | **Observations cliniques** | **Temps nécessaire à la guérison** |
|---|---|---|---|
| Chien n°1 | 0,5 mg/kg | Une amélioration est visible dès la première prise | Guérison en 21 jours |
| Chien n° 2 | 0,5 mg/kg | Une amélioration est visible dès la première prise | Guérison en 21 jours |
| Chien n°3 | 0,5 mg/kg | Une amélioration est visible dès la première prise | Guérison en 60 jours |
| Chien n° 4 | 0,5 mg/kg | Amélioration après une semaine | Guérison en 60 jours |
| Chien n° 5 | 0,25 mg/kg | Les modifications sont peu visibles, la malpropreté n'est pas jugulée, pas d'effets secondaires | Amélioration sans guérison |
| Chien n° 6 | 0,25 mg/kg | Les modifications sont peu visibles, la malpropreté n'est pas jugulée, pas d'effets secondaires | Amélioration sans guérison |

Sur le plan clinique, nous avons observé des troubles dominés par la production de réponses émotionnelles disproportionnées, fréquentes et prolongées. Ces manifestations tendent, pour celles-ci qui ont une expression volontaire, à se déréguler et à évoluer vers des stéréotypies. Ainsi, nous observons des activités de léchage perdant progressivement leur interruption spontanée pour aboutir à un léchage stéréotypé envahissant progressivement les périodes de veille. Il en est de même des boulimies et potomanies, mais aussi des réponses agressives à des composantes émotionnelles... Des troubles du sommeil correspondant à un avancement du sommeil paradoxal aboutissant à des réveils brutaux au cours des 90 premières minutes de sommeil et associés à des phases d'inquétude et d'agitation à l'approche du coucher, sont d'observation systématique chez ces animaux. Cette typologie précise nous a amenés à définir une échelle d'évaluation chiffrée de l'état thymique des animaux que nous appelons l'ETEC (Evaluation des Troubles Emotionnels des Chiens) qui nous servira d'outil de suivi des animaux traités et permettra une quantification de leur guérison.
- ETEC ≤ 20 Animal normal
- ETEC > 20 Animal atteint de troubles du comportement avec altération de l'humeur.

La grille d'évaluation est jointe en annexe de la description.

### 2. Etude clinique

Dans le tableau II suivant sont résumés les résultats d'une étude réalisée sur 25 chiens de races diverses. La valeur de l'ETEC a été mesurée au premier jour (J = 0), au 30ème jour (J = 30) et au 60ème jour (J = 60) du traitement.

**TABLEAU II**

| **ANIMAL** | | **E T E C** | | | **DIAGNOSTIC** |
|---|---|---|---|---|---|
| **Nom ou Race** | **Age** | **J=0** | **J=30** | **J=60** | |
| Caniche noir | 17 ans | 42 | 15 | 11 | Dysthymie |
| Setter irlandais | 05 ans | 40 | 14 | 11 | Dysthymie |
| Croisé pinscher | 06 ans | 37 | 22 | 20 | Dysthymie |
| Caniche | 07 ans | 26 | 14 | 14 | Dysthymie |
| Berger allemand | 08 ans | 36 | 16 | 15 | Dépression d'involution |
| Cocker | 12 ans | 43 | 23 | 18 | Agressif dominant |
| Croisé berger | 2,5 ans | 39 | 23 | - | Dominant + stéréotypie de mordillement |
| Dark | 05 ans | 24 | 24 | 13 | Hyperactivité + anxiété intermittente |
| Pinscher | 01 an | 38 | 21 | 19 | Hyperactivité + hyperattachement |
| Labrador | 10 mois | 44 | 18 | 14 | Hyperactivité |
| Berger des Pyrénées | 03 ans | 43 | 19 | 18 | Syndrome de privation |
| Virgile | 08 ans | 22 | 19 | 19 | Anxiété de séparation |
| Christopher | 06 ans | 25 | 18 | - | Anxiété de séparation |
| Hatoll | 01 an | 26 | 18 | 13 | Anxiété de séparation |
| Libre | 06 ans | 26 | 20 | 19 | Anxiété globale |
| Elia | 4,5 ans | 25 | 25 | 10 | Anxiété globale |
| Honorine | 1,5 ans | 22 | 21 | 15 | Anxiété globale |
| Scarlett | 05 ans | 21 | 22 | 15 | Anxiété paroxystique |
| Montagne des Pyrénées | 05 ans | 30 | 24 | 18 | Polyphobie - tournis stéréotypie |
| Léonberg | 10 mois | 24 | 21 | 18 | Polyphobie + anxiété permanente |
| Berger allemand | 03 ans | 22 | 22 | 15 | Phobie humaine |
| Berger allemand | 02 ans | 24 | 20 | | Phobie urbaine + sociopathie |
| Doberman | 01 an | 22 | 17 | 16 | Phobie urbaine + anxiété intermittente |
| Croisé berger | 05 ans | 30 | 32 | 17 | Sociopathie instrumentale |
| Caniche | 10 ans | 29 | 22 | | Sociopathie instrumentale + tournis |

Cette étude met en évidence l'efficacité de l'utilisation de la sélégiline selon la présente invention. Les animaux, presque sans exception, ont atteint on fin de traitement un ETEC inférieur à 20 caractérisant un état normal.

En conséquence, la sélégiline peut être utilisée dans les troubles du comportement avec altération de l'humeur regroupant les pathologies suivantes :

| | |
|---|---|
| - dysthymie | - anxiété intermittente |
| - dépression d'involution | - anxiété paroxystique |
| - agressivité par dominance | - sociopathie instrumentale |
| - hyperactivité | - stéréotypie (tournis/léchage) |
| - syndrome de privation | - phobie (humaine/urbaine) |
| - anxiété de séparation | - troubles de socialisation |

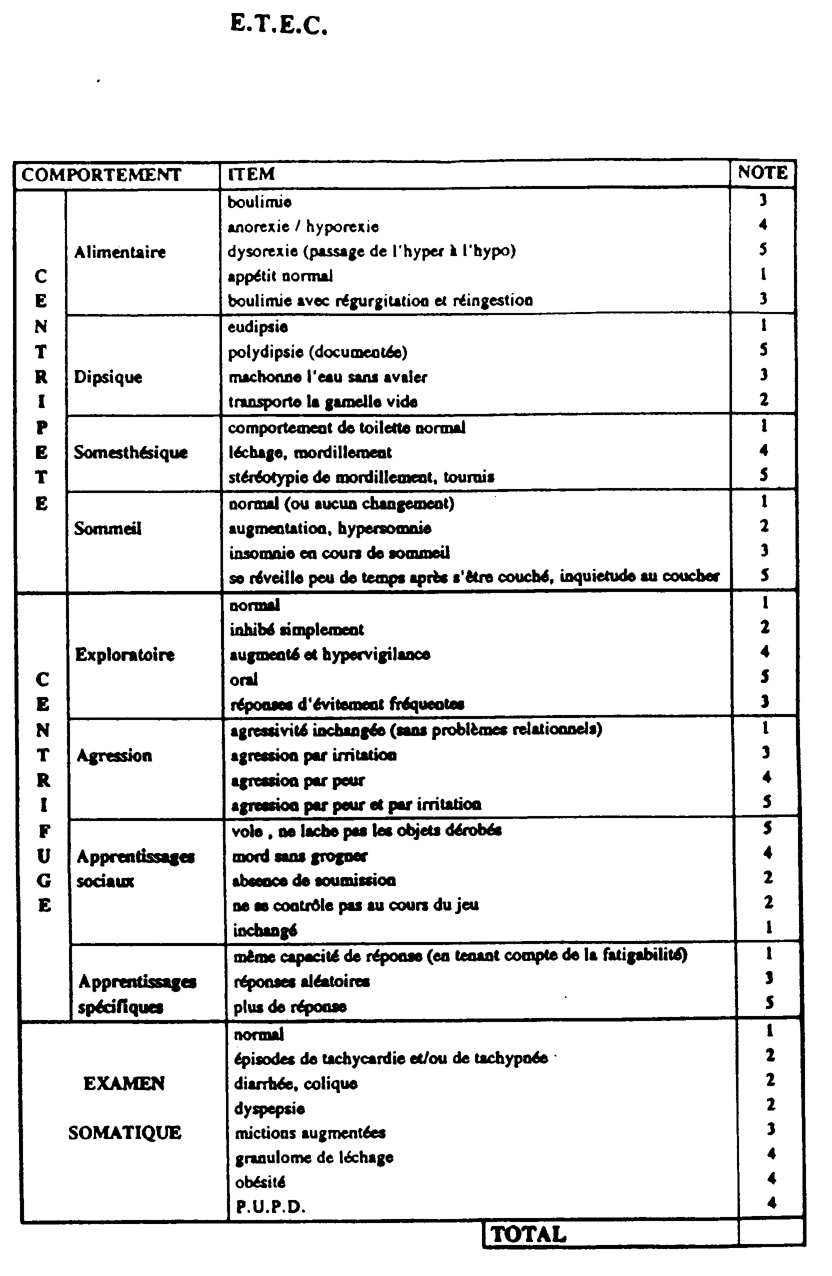

## Revendications

1. Utilisation de la sélégiline, de son racémique, de l'isomère lévogyre ou de leurs mélanges en proportions quelconques et de leurs sels pharmaceutiquement acceptables pour la préparation d'une composition pharmaceutique destinée au traitement des troubles du comportement avec altération de l'humeur chez le chien ou le chat,
- par voie orale à une dose journalière de 0,25 à 1 mg/kg de poids corporel de l'animal ; ou
- par voie sous-cutanée à une dose journalière de 0,25 à 4 mg/kg de poids corporel de l'animal.

2. Utilisation selon la revendication 1, du chlorhydrate de sélégiline.

3. Utilisation selon l'une quelconque des revendications précédentes en vue d'une administration orale.

4. Utilisation selon l'une quelconque des revendications précédentes caractérisée en ce que les troubles du comportement avec altération de l'humeur se traduisent par l'un au moins des symptômes suivants:
- boulimie avec ou sans régurgitation et réingestion;
- anorexie;
- dysorexie;
- eudipisie;
- polydipsie;
- mâchonnement de l'eau sans ingestion de celle-ci;
- rituels de mordillement;
- léchages anormaux;
- malpropreté urinaire ou fécale; ou
- sommeil perturbé.

5. Utilisation selon l'une quelconque des revendications 1 à 3, caractérisée en ce que les troubles du comportement avec altération de l'humeur sont observés dans le cadre de l'une ou plusieurs des pathologies suivantes:
- dysthymie;
- dépression d'involution ;
- agressivité par dominance ;
- hyperactivité;
- syndrome de privation;
- anxiété de séparation;
- anxiété intermittente;
- anxiété paroxystique;
- sociopathie instrumentale;
- stéréotypie (tournis/léchage);
- phobie (humaine/urbaine); ou
- troubles de socialisation.

## Claims

1. Use of selegilin, the racemate, the laevorotatory isomer thereof or mixtures thereof in any proportions and the pharmaceutically acceptable salts thereof for preparing a pharmaceutical composition intended for the treatment of behavioural disorders with mood changes in dogs or cats,
- by oral route in a daily dose of from 0.25 to 1 mg/kg of the body weight of the animal; or
- by subcutaneous route in a daily dose of from 0.25 to 4 mg/kg of the body weight of the animal.

2. Use according to claim 1 of selegilin hydrochloride.

3. Use according to any one of the preceding claims for oral administration.

4. Use according to any one of the preceding claims, characterised in that the behavioural disorders with mood changes are demonstrated by at least one of the following symptoms:
- bulimia with or without regurgitation and reingestion;
- anorexia;
- dysorexia;
- eudipsia;
- polydipsia;
- masticating water without ingesting it;
- biting rituals;
- abnormal licking;
- urinary or faecal soiling; or
- disturbed sleep.

5. Use according to any one of claims 1 to 3, characterised in that the behavioural disorders with mood changes are observed within the scope of one or more of the following pathologies:
- dysthymia;
- withdrawn depression;
- dominant aggression;
- hyperactivity;
- deprivation syndrome;
- separation anxiety;
- intermittent anxiety;
- paroxystic anxiety;
- instrumental sociopathy;
- stereotypical behaviour (circling/licking);
- phobia (human/urban) or
- socialisation problems.

## Patentansprüche

1. Verwendung von Selegilin, seines Racemats, seines linksdrehenden Isomeren oder Mischungen davon in beliebigen Verhältnissen und seiner pharmazeutisch annehmbaren Salze für die Herstellung einer pharmazeutischen Zubereitung zur Behandlung von Verhaltensstörungen mit Änderung der Stimmung bei Hund oder Katze,
- auf oralem Wege in einer täglichen Dosis von 0,25 bis 1 mg/kg Körpergewicht des Tieres; oder
- auf subkutanem Wege in einer täglichen Dosis von 0,25 bis 4 mg/kg Körpergewicht des Tieres.

2. Verwendung nach Anspruch 1 von Selegilin-Hydrochlorid.

3. Verwendung nach irgendeinem der vorhergehenden Ansprüche im Hinblick auf eine orale Verabreichung.

4. Verwendung gemäß irgendeinem der vorhergehenden Ansprüche, dadurch gekennzeichnet, daß die Verhaltensstörungen mit Änderung der Stimmung sich in mindestens einem der folgenden Symptome manifestieren:
- Bulimie mit oder ohne Wiederverschlucken und Wiederaufnahme;
- Anorexie;
- Dysorexie;
- Eudipsie;
- Polydipsie;
- Kauen von Wasser ohne seine Aufnahme;
- Rituale des Knabberns;
- abnormales Lecken;
- Urin- oder Fäzes-Unreinheit; oder
- gestörter Schlaf.

5. Verwendung nach irgendeinem der Ansprüche 1 bis 3, dadurch gekennzeichnet, daß die Verhaltensstörungen mit Änderung der Stimmung im Rahmen eines oder mehrerer der folgenden pathologischen Zustände beobachtet werden:
- Dysthimie;
- Involutions-Depression;
- Dominanz-Aggressivität;
- Hyperaktivität;
- Enzugssyndrom;
- Trennungsangst;
- wechselnde Angst;
- Angstanfälle;
- instrumentale Soziopathie;
- Stereotypie (Drehen/Lecken);
- Phobie (human/urban); oder
- Sozialisierungsstörungen.
